# EUROPÄISCHE PATENTSCHRIFT

(11) **EP 1 761 477 B1**
(45) Veröffentlichungstag und Bekanntmachung des Hinweises auf die Patenterteilung: **01.12.2010**
(21) Anmeldenummer: 05754875.2
(22) Anmeldetag: 16.06.2005
(51) Int. Cl.: C07C 209/16, C07C 211/04

(54) **VERFAHREN ZUR KONTINUIERLICHEN SYNTHESE VON METHYLAMINEN**
METHOD FOR THE CONTINUOUS SYNTHESIS OF METHYLAMINES
PROCEDE POUR REALISER LA SYNTHESE CONTINUE DE METHYLAMINES

(30) Priorität: 18.06.2004 DE 102004029544; 09.06.2005 DE 102005026515
(43) Veröffentlichungstag der Anmeldung: 14.03.2007
(73) Patentinhaber: BASF SE, 67056 Ludwigshafen (DE)
(72) Erfinder: BOSCH, Marco, 68159 Mannheim (DE); EBERHARDT, Jan, 68167 Mannheim (DE); RÖTTGER, Roderich, 68165 Mannheim (DE); KRUG, Thomas, 67550 Worms (DE); MELDER, Johann-Peter, 67459 Böhl-Iggelheim (DE)
(86) Internationale Anmeldenummer: PCT/EP2005/006480
(87) Internationale Veröffentlichungsnummer: WO 2005/123658

(56) Entgegenhaltungen:
- EP-A- 0 632 012
- US-A1- 2001 002 383

## Beschreibung

Die vorliegende Erfindung betrifft ein Verfahren zur kontinuierlichen Synthese von Methylaminen durch Umsetzung von Methanol und/oder Dimethylether mit Ammoniak in Gegenwart eines Heterogenkatalysators, wobei als Katalysator ein Formkörper enthaltend ein mikroporöses Material und mindestens ein Bindemittel eingesetzt wird.

Monomethylamin (MMA) ist ein Zwischenprodukt, welches in der Synthese von Pharmazeutika (z.B. Theophyllin), Pestiziden (Carbaryl, Metham Natrium, Carbofuran), Tensiden, photographischen Entwicklern, Sprengstoffen und Lösungsmitteln wie *N-*Methyl-2-pyrrolidon (NMP) eingesetzt wird.

Dimethylamin (DMA) ist ebenfalls ein synthetisches Zwischenprodukt. Beispiele für Produkte auf Dimethylamin-Basis sind Fungizide und Vulkanisationsbeschleuniger (Zink-bis-Dimethyldithiocarbamat) (Ziram), Tetramethyl-thioperoxydikohlensäurediamid (TMTD), Tetramethyl-thiokohlensäure-diamid (MTMT), das Treibmittel 1,1-Dimethylhydrazin, verschiedene Pharmazeutika, Monomere wie z.B. Dimethylaminoethylmethacrylat, Lösungsmittel (*N,N*-Dimethylformamid, *N,N*-Dimethylacetamid), Katalysatoren [z. B. 2,4,6-Bis[(dimethylamino)methyl]phenol (DMP 30)], das Insektizid Dimefax, Tenside und Ionenaustauschharze.

Trimethylamin (TMA) wird bei der Herstellung von Cholinsalzen, kationischen Stärken, Desinfektionsmitteln, Flotationsmitteln, Süßstoffen und Ionenaustauschharzen eingesetzt.

Die klassische Synthese von Monomethylamin (MMA), Dimethylamin (DMA) und Trimethylamin (TMA) erfolgt aus Ammoniak und Methanol in der Gasphase an amorphem nicht-formselektivem Silica-Alumina (Mischformen von Aluminium- und Siliziumoxid) bei Drücken von 10 bis 50 bar. Bei der Anwendung von höheren Temperaturen (350 bis 475°C) stellt sich an diesen heterogen Katalysatoren das thermodynamische Gleichgewicht ein oder wird näherungsweise erreicht, wenn die Verweilzeit im Reaktor bei gegebenem Druck und gegebener Zulauftemperatur hinreichend ist. Charakteristisch für diese "Gleichgewichtskatalysatoren" ist ein Anteil an Trimethylamin im Reaktoraustrag, bezogen auf die Summe von Monomethylamin, Dimethylamin und Trimethylamin, von 35 bis 60 Gewichtsprozent. Die Produktverteilung ist abhängig von der Temperatur und vom N/C-Verhältnis. Der Anteil von Trimethylamin am Produktgemisch lässt sich dabei reduzieren, wenn ein höherer Überschuss an Ammoniak (größeres N/C-Verhältnis) im Reaktionsgemisch enthalten ist. Wenn der Anteil an Monomethylamin und/oder Dimethylamin am gewünschten, nach der bekannten Aufarbeitung entnommenen Produktgemisch größer ist als es dem Reaktoraustrag entspricht, müssen sowohl das überschüssige Trimethylamin als auch das nicht umgesetzte Ammoniak zurück in den Reaktor gefahren werden, wobei große Ammoniak- und Trimethylamin-Kreisläufe entstehen.

Der weltweite Trimethylaminverbrauch liegt bezogen auf die Gesamtmenge der Methylamine bei 10 bis 20 Gewichtsprozent. Es ist wünschenwert, den Anteil an DMA und MMA ohne Rückführung des Reaktionsgemisches zu erhöhen. Dies gelingt durch die Verwendung von formselektiven Katalysatoren bei Temperaturen von 250 bis 400°C. Man erhält ein Produktgemisch, das vorwiegend aus Dimethyl- und Monomethylamin besteht und nur wenig Trimethylamin enthält.

Die formselektiven Eigenschaften werden hervorgerufen, wenn der Porendurchmesser des Molekularsiebs kleiner ist als der kinetische Durchmesser des Trimethylamins von ca. 6,1 Å (Stud. Surf. Sci. Catal. 1993, 75, 1273 -1283). Bevorzugt eingesetzt werden Molekularsiebe mit einem Porendurchmesser kleiner 5,5 Å, besonders bevorzugt kleiner 5,0 Å.

Molekularsiebe mit Porendurchmesser von 6,1 Å und größer können durch chemische Modifikationen zu formselektiven Materalien umgewandelt werden. Die Modifikationen bewirken eine Reduktion der effektiven Porendurchmesser auf kleiner 5,5 Å, bevorzugt kleiner 5,0 Å. Die Modifikationen umfassen ein partieller Ionenaustausch mit Alkali-, Erdalkali-, Übergangsmetall- und/oder Lanthanidionen, eine Behandlung des Molekularsiebs mit silizium- und/oder phosphorhaltigen Substanzen und/oder eine Behandlung des Molekularsieb mit Wasserdampf.

Besonders zu erwähnen ist die Behandlung von Molekularsieben und insbesondere von Zeolithen mit siliziumhaltigen Verbindungen in der Gasphase.

Für diese Gasphasen-Methode werden bevorzugt Mordenitkatalysatoren mit Siliciumverbindungen wie SiCl₄ (JP 262540/1991; J. Catal. 1991, 131, 482; US 5,137,854) und Si(OMe)₄ bzw. Si(OEt)₄ (Shokubai, 1987, 29, 322, J. Chem. Soc., Chem. Commun. 1982, 819) umgesetzt. Andere Beispiele mit Katalysatoren auf Basis Chabazit, Erionit, ZK-5 und Rho und ihre Behandlung mit silizium-, aluminium-, bor- und/oder phosphorhaltigen Verbindungen sind in JP 254256/1986 und US 4,683,334 beschrieben.

Die Silylierung von Mordenitkatalysatoren in der Flüssigphase mit Tetraalkoxysilanen ist in EP-A-593 086 beschrieben.

Bekannt ist ebenfalls, dass nach der Silylierung der behandelte Zeolith einer Wärmebehandlung (= Kalzinierschritt) unterzogen wird (J. Chem. Soc., Faraday Trans. 1984, 180, 3135; EP-A-593 086).

Sowohl die Flüssig- als auch die Gasphasensilylierung haben den Nachteil, dass die bei der Reaktion entstehenden Produkte (HCl bei SiCl₄ bzw. ROH bei Si(OR)₄) von dem Zeolithpulver getrennt werden müssen.

Im Falle der Silylierung mit SiCl₄ wird eine technische Anwendung erschwert durch die korrosiven Eigenschaften des dabei entstehenden HCl.

Nach der Behandlung mit SiCl₄ sind ein oder mehrere Schritt(e) notwendig, um eine SiO₂-Schicht zu erzeugen. Bei der Silylierung des Zeolithpulvers in der Flüssigphase mit Tetraalkoxysilanen Si(OR)₄ muss neben dem dabei entstehenden Abspaltungsprodukt ROH auch das Lösungsmittel (üblicherweise C₁₋₆-Alkohole, C₅-C₈-(Cyclo)alkane und/oder Toluol) noch entfernt und das Pulver nachträglich getrocknet werden, bevor es in dem Verformungsschritt eingesetzt werden kann.

Für beide Silylierungs-Methoden sind also zusätzliche Arbeitsschritte notwendig um einen silylierten Katalysator herzustellen. Diese zusätzlichen Arbeitsschritte und/oder die bei der Silylierung entstehenden Produkte können dafür sorgen, dass eine technische Umsetzung solcher Methoden aus Kostensicht und/oder aus praktischen Gründen unwirtschaftlich ist.

Die Verwendung von kollodialem Silica als SiO₂-Binder zur Herstellung von Katalysatorformkörpern ist in
"Catalyst Support and Supportes Catalysts" (A.B. Stiles), 1987, Kapitel 1, auf den Seiten 1 bis 9 und in Kapitel 3 auf den Seiten 57 bis 62, in
"Applied Heterogeneous Catalysis - Design, Manufacture, Use Of Solid Catalysts" (J.-F. Lepage, J. Cosyns, P. Courty, E.B. Miller), 1987, Kapitel 5, auf den Seiten 75 bis 123, in
"Heterogeneous Catalysis In Industrial Practice" (C.N. Satterfield), 2. Ausgabe, 1991, Kapitel 4, auf den Seiten 87 bis 130 und speziell auf Seite 121, sowie in
"Studies in Surface Science and Catalysis" (E.B.M. Doesburg, J.H.C. Hooff), 1993, Kapitel 8 auf den Seiten 309 bis 332,
beschrieben.

Der Einsatz von kollodialen Silica und speziell Ludox® AS40 der Firma DuPont als SiO₂-Binder für die Verformung von ZSM-5-Pulver wird in US 6,077,984 beschrieben.

Die ältere deutsche Patentanmeldung Nr. 10356184.6 vom 02.12.03 (BASF AG) betrifft ein bestimmtes zeolithisches Material vom Pentasil-Strukturtyp mit einem Alkali- und Erdalkalimetallgehalt ≤ 150 ppm und einer Spezifikation bezüglich kugelförmiger Primärpartikel, das mit kolloidalem Silica als Bindemittel verformt ist, und seine Verwendung als Katalysator.

Gemäß WO-A-01/23089 werden Katalysatorformkörper erhalten, bestehend aus einem ZSM-5-Pulver und einem SiO₂-Binder mit Schneidhärten größer 1 kg. Als SiO₂-Bindemittel werden kolloidale Silica eingesetzt.

WO-A-03/092887 (DE-A1-102 19 879) (BASF AG) betrifft ein Verfahren zur Herstellung eines Katalysatorträgers bei dem man Zirkoniumdioxidpulver mit einem Bindemittel zu Formkörper formt, trocknet und calciniert, wobei das Bindemittel eine monomere, oligomere oder polymere Organosiliciumverbindung ist. Gegenstand der Anmeldung ist auch der so hergestellte Katalysatorträger selbst, ein Katalysator, der diesen Träger enthält sowie dessen Verwendung als Dehydrierkatalysator.

Die Verformung von kristallinen Molekularsieben mit Übergangsmetalloxiden, Schichtsilikaten und Tonen und die Verwendung der Formkörper für die formselektive Herstellung von Methylaminen aus Methanol und Ammoniak ist in der Patentanmeldung JP 2000-005604 (= EP-A-967 011) beschrieben. Als kristalline Molekularsiebe werden Silicoalumophosphate, Mordenit und Chabazit bevorzugt. Als Binder werden Zirkonoxid, Yttriumoxid und Titanoxid bevorzugt, der Anteil beträgt bevorzugt zwischen 1 und 20 Gew.-%.

In der Offenlegungsschrift CN-A-1 095 645 wird die Verwendung von siliziumhaltigen anorganischen Substanzen als inertes Bindermaterial für die Herstellung von formselektiven Molekularsiebkatalysatoren, die für die Herstellung von Methylaminen eingesetzt werden, beschrieben. Das Gewichtsverhältnis an Binder (bezogen auf SiO₂ im fertigen Strang) liegt zwischen 30 und 70 Gew.%. Zu dem Formungsverfahren können Geliermittel wie Ammoniumnitrat, Natriumnitrat oder Kaliumnitrat sowie Porenerweiterungsmittel wie Tenside oder Pflanzenstärken zugesetzt werden. Der Anteil an Porenerweiterungsmittel liegt bevorzugt bei kleiner 10 Gewichtsprozent, bevorzugt zwischen 3 und 7 Gewichtsprozent.

EP 632012-A2 betrifft Verfahren zur Herstellung von Methylaminen aus Methanol oder Dimethylether und Ammoniak unter Verwendung von mikroporösen Zusammensetzungen als Katalysator.

Der vorliegenden Erfindung lag die Aufgabe zugrunde, ein verbessertes wirtschaftliches Verfahren zur Herstellung von Methylaminen (MMA, DMA, TMA; insbesondere DMA) aufzufinden. Das Verfahren sollte ein oder mehrere Nachteile der Verfahren des Stands der Technik überwinden. Das Verfahren sollte, insbesondere bei der Umsetzung von Methanol mit Ammoniak, eine hohe Selektivität für Dimethylamin (DMA) aufweisen, welche im Besonderen höher ist als im thermodynamischen Gleichgewicht der Methylamine.

Demgemäß wurde ein Verfahren zur kontinuierlichen Synthese von Methylaminen durch Umsetzung von Methanol und/oder Dimethylether mit Ammoniak in Gegenwart eines Heterogenkatalysators gefunden, welches dadurch gekennzeichnet ist, dass als Katalysator ein Formkörper enthaltend ein mikroporöses Material und mindestens eine siliciumorganische Verbindung als Bindemittel, herstellbar durch ein Verfahren umfassend die Schritte
(I) Herstellen eines Gemischs, enthaltend das mikroporöse Material, das Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers
eingesetzt wird.

Überraschenderweise wurde gefunden, dass die Verformung eines mikroporösen Materials, insbesondere eines zeolithischen Materials (z.B. eines Zeolithpulvers) mit einer siliziumorganischen Verbindung (z.B. einem Silikon) als Bindemittel gelingt und zu Formkörpern führt, deren mechanische Eigenschaften (insbesondere Schneidhärte) den mit kolloidalem Silica verformten Formkörpern deutlich überlegen ist ohne, dass diese verbesserte mechanische Stabilität sich negativ auf die Selektivität und/oder Aktivität des Formkörpers als Katalysator bei der Herstellung von Methylaminen auswirkt.

Der im erfindungsgemäßen Verfahren als Katalysator eingesetzte Formkörper weist eine verbesserte mechanische Stabilität, z.B. gemessen als Schneidhärte (in Newton (N)), z.B. eine Schneidhärte von größer oder gleich 10 N, auf.

Darüber hinaus ermöglichen die als Katalysatoren bei der formselektiven Synthese von Methylaminen eingesetzten Formkörper bessere Umsätze und Raum-Zeit-Ausbeuten, höhere Selektivitäten und längere Standzeiten, z.B. durch verminderte Neigung zur Verkokung.

Im erfindungsgemäßen Verfahren beträgt vorteilhaft der Anteil an TMA im Methylamine-Produktgemisch kleiner 10 Gew.%, insbesondere kleiner als 5 Gew.-%, jeweils bezogen auf das Gewicht aller drei Methylamine (MMA, DMA, TMA).

Zum Katalysatorformkörper, der im erfindungsgemäßen Verfahren eingesetzt wird:
Die siliciumorganische Verbindung als Bindemittel (Schritt I)

Als siliciumorganisches Bindemittel geeignet sind monomere, oligomere oder polymere Silane, Alkoxysilane, Acyloxysilane, Oximinosilane, Halogensilane, Aminoxysilane, Aminosilane, Amidosilane, Silazane oder Silikone, wie sie beispielsweise in Ullmann's Encyclopedia of Industrial Chemistry, Vol. A24, auf den Seiten 21 bis 56, und in Lehrbuch der Anorganischen Chemie (A.F. Holleman, E. Wiberg), 100. Auflage, Kapitel 2.6.5, auf den Seiten 786 bis 788 beschrieben sind. Insbesondere gehören dazu die monomeren Verbindungen der nachstehenden Formeln (A) bis (F):

(Hal)ₓSiR₄₋ₓ (A)

(Hal)ₓSi(OR)₄₋ₓ (B)

(Hal)ₓSi(NR¹R²)₄₋ₓ (C)

RₓSi(OR¹)₄-ₓ (D)

RₓSi(NR¹R²)₄₋ₓ (E)

(RO)ₓSi(NR¹R²)₄₋ₓ (F)

worin

Hal unabhängig voneinander Halogen (F, Cl, Br oder I, insbesondere Cl),
R, R¹, R² unabhängig voneinander H oder einen gegebenenfalls substituierten Alkyl-, Alkenyl-, Alkinyl-, Cycloalkyl-, Cycloalkenyl-, Arylalkyl- oder Arylrest, und
x eine ganze Zahl im Bereich von 0 bis 4
bedeuten.

Bei den Alkylresten sind C₁₋₆-Alkylreste bevorzugt. Sie können linear oder verzweigt sein. Bevorzugte Beispiele sind Methyl, Ethyl, n-Propyl, n-Butyl, sec.-Butyl oder tert.-Butyl, speziell Methyl oder Ethyl.

Als Arylreste sind C₆₋₁₀-Arylreste bevorzugt, beispielsweise Phenyl. Bevorzugte Arylalkylreste sind C₇₋₂₀-Arylalkylreste, insbesondere Benzyl. Bevorzugte Alkenylreste sind C₂₋₆-Alkenylreste, insbesondere Vinyl oder Allyl. Als Alkinylreste sind C₂₋₆-Alkinylreste bevorzugt, beispielsweise Ethinyl oder Propargyl. Bei den Acylresten sind C₂₋₆-Acylreste bevorzugt, insbesondere ein Acetyl. Bevorzugte Cycloalkylreste sind C₅₋₈-Cycloalkylreste, insbesondere Cyclopentyl oder Cyclohexyl.
Bevorzugte Cycloalkenylreste sind C₅₋₈-Cycloalkenylreste, beispielsweise 1-Cyclopentenyl oder 1-Cyclohexenyl.

Beispiele für geeignete Organosiliciumverbindungen der Formel (A) sind SiCl₄, Me-SiCl₃, Me₂SiCl₂ und Me₃SiCl.

### Beispiele für geeignete Organosiliciumverbindungen der Formel (B) sind Si(OMe)₄, ClSi(OMe)₃, Cl₂Si(OMe)₂, Cl₃SiOMe. (Me = Methyl)

Beispiele für geeignete Organosiliciumverbindungen der Formel (C) sind Si(NMe₂)₄, ClSi(NMe₂)₃, Cl₂Si(NMe₂)₂, Cl₃SiNMe₂.

Geeignete Organosiliciumverbindungen der Formel (D) sind beispielsweise Si(OEt)₄, MeSi(OEt)₃, Me₂Si(OEt)₂ und Me₃Si(OEt).

Geeignete Verbindungen der Formel (E) sind beispielsweise Me₃Si(N(Me)COMe) und Me₃Si(N(Me)COCH₂C₆H₅).

Eine geeignete Verbindung der Formel (F) ist beispielsweise (MeO)₃Si(NMe₂).

Bevorzugt wird als Bindemittel ein cyclisches Silikon der Formel [-SiO(OR)(R')-]ₓ oder ein lineares Silikon der Formel RO-[SiO(OR)(R')-]ₓ-R oder ein Gemisch dieser Silikone eingesetzt, wobei R und R' (unabhängig voneinander) C₁₋₆-Alkylgruppen (wie oben definiert), insbesondere Methyl, Ethyl, und x eine Zahl im Bereich von 2 bis 50, insbesondere eine Zahl im Bereich von 3 bis 20, bedeuten.

Ganz besonders bevorzugte siliciumorganisches Bindemittel sind Methylsilikone, beispielsweise die Silres®-Marken der Fa. Wacker, z.B. Sitres® MSE100.

Für die Verformung werden halogenfreie siliciumorganische Bindemittel bevorzugt, um Korrosion während der Präparation der Formkörper bzw. beim Einsatz der Formkörper in der katalytischen Umsetzung zu vermeiden.

Die als Bindemittel eingesetzten Organosiliciumverbindungen sind bevorzugt unter Normalbedingungen flüssig oder werden als Lösung in einem bevorzugt nicht-polaren organischen Lösungsmittel wie Hexan, Toluol und/oder Cyclohexan eingesetzt. Dadurch wird die hochoberflächige mikroporöse Aktivkomponente beim Vermischen gleichmäßig mit der Organosiliciumverbindung benetzt. Beim Calcinieren der Katalysatorformkörper verbrennen die organische Reste des siliciumorganischen Bindemittels. Dabei wird SiO₂ gebildet, welches im Formkörper sehr fein verteilt vorliegt. Daraus resultiert eine hohe Bindungsverstärkung zwischen den Primärpartikeln der mikroporösen Aktivkomponente und eine sehr gute mechanische Stabilität der erhaltenen Katalysatorformkörper. Durch die Verbrennung der organischen Reste des siliciumorganischen Bindemittels entstehen zusätzliche Poren. Diese Poren sind aufgrund der gleichmäßigen Verteilung des siliciumorganischen Bindemittels im Formkörper ebenfalls sehr gleichmäßig verteilt. Dadurch wird die Gesamtporosität des Katalysatorsträgers erhöht.

Bevorzugt wird durch die Calcinierung der Formkörper gemäß Schritt V mindestens 80 Gew.%, insbesondere mindestens 95 Gew.%, der siliciumorganischen Verbindung in hochdisperses SiO₂ umgewandelt. Der Gewichtsanteil des so gebildeten hochdispersen SiO₂ im fertigen Katalysatorformkörper liegt vorzugsweise im Bereich von 5 und 70 Gew.-%, insbesondere im Bereich von 10 und 50 Gew.-%, ganz besonders im Bereich von 10 und 30 Gew.-%.

### Das mikroporöse, insbesondere zeolithische Material (Schritt I)

Das mikroporöse Material ist bevorzugt ein Molekularsieb mit einem Porendurchmesser kleiner 5 Å. Dabei sind im einzelnen Molekularsiebe der Typen mit röntgenographischer Zuordnung zur ABW-, ACO-, AEI-, AEN-, AFN-, AFT-, AFX-, ANA-, APC-, APD-, ATN-, ATT-, ATV-, AWO-, AWW-, BIK-, BRE-, CAS-, CDO-, CHA-, DDR-, DFT-, EAB-, EDI-, ERI-, ESV-, GIS-, GOO-, ITE-, ITW-, JBW-, KFI-, LEV-, LTA-, MER-, MON-, MOR-, MTF-, PAU-, PHI-, RHO-, RTE-, RTH-, RUT-, SAS-, SAT-, SAV-, THO-, TSC-, UEI-, UFI-, VNI-, YUG-, ZON-Struktur sowie zu Mischstrukturen aus zwei oder mehr der vorgenannten Strukturen zu nennen. Bevorzugt ist das Molekularsieb ein kristallines Alumosilikat (= zeolithisches Material), ein kristallines Silicoalumophosphat und/oder ein kristallines Alumophosphat. Besonders bevorzugt sind kristalline Alumosilikate, speziell Zeolithe.

Zeolithe sind kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier, D.H. Olson und Ch. Baerlocher, "Atlas of Zeolithe Structure Types", Elsevier, 5. Auflage, Amsterdam 2001.

Wahlweise können auch Molekularsiebe mit einem Porendurchmesser größer 5 Å eingesetzt werden, deren effektive Porendurchmesser durch eine oder mehrere chemische Modifikationen auf kleiner 5 Å gebracht wurde. Dabei sind im einzelnen Molekularsiebe der Typen mit röntgenographischer Zuordnung zur BEA-, EUO-, FAU-, FER-, HEU-, MEL-, MFI-, MOR-, MWW- und OFF-Struktur sowie zu Mischstrukturen aus zwei oder mehr der vorgenannten Strukturen zu nennen. Bevorzugtes Molekularsieb ist ein kristallines Alumosilikat (= zeolithisches Material), ein kristallines Silicoalumophosphat und/oder ein kristallines Alumophosphat. Besonders bevorzugt sind kristalline Alumosilikate, speziell Zeolithe.

Eine allgemeine Beschreibung der chemischen Modifikationen ist in Kapitel 3 und insbesondere in Kapitel 3.1, 3.3 und 3.5 von "Catalysis and Zeolites; Fundamentals and Applications" (Springer Verlag, Heidelberg, 1999, S. 81-179) beschrieben. Die Modifikationen umfassen den partiellen Austausch des Molekularsiebs mit Alkali-, Erdalkali-, Übergangsmetall- und/oder Lanthanidionen analog EP-A-0125 616, eine Behandlung des Molekularsiebs mit bor- und aluminiumhaltigen Verbindungen analog WO-A-99/02483, mit siliziumhaltigen Verbindungen analog JP-B2-300 1162, EP-A-593 086 und KR 2002/0047532 oder phosphorhaltigen Verbindungen analog WO-A-99/02483 und WO-A1-2004/002937, sowie eine Behandlung des Molekularsiebs mit Wasser(dampf) analog EP-A-0130 407. Die Modifikationen können mehrfach wiederholt und miteinander kombiniert werden.

Von den oben erwähnten Molekularsieben werden Zeolithe der Typen mit röntgenographischer Zuordnung zur CHA-, ERI-, EUO-, FAU-, FER-, HEU-, KFI-, LEV-, LTA-, MEL-, MFI-, MOR-, OFF-, PHI-, RHO-Struktur sowie zu Mischstrukturen aus zwei oder mehr der vorgenannten Strukturen bevorzugt eingesetzt.

Als Zeolith wird Mordenit mit small-port Eigenschaften wie beschrieben in "Catalysis and Zeolites; Fundamentals and Applications" (Springer Verlag, Heidelberg, 1999, S. 41 - 42) besonders bevorzugt. Das small-port Mordenit kann synthetisch, nach dem Fachmann bekannten Methoden hergestellt werden oder als natürliches Produkt eingesetzt werden.

Das mikroporöse Material gemäß (I) liegt bevorzugt zumindest teilweise in der H⁺- und/oder NH₄⁺-Form vor. Besonders bevorzugt wird das mikroporöse Material gemäß (I) mindestens teilweise in der H⁺-Form und insbesondere bevorzugt für mehr als 60 % (bezogen auf die Zahl der Brönstedzentren im Zeolith) in der H⁺-Form eingesetzt.

Was die Primärpartikel des mikroporösen Materials gemäß (I) anbelangt, so sind Partikelgrößen von weniger als 10 µm, besonderes von weniger als 5 µm und insbesondere von weniger 2 µm bevorzugt. Die Partikelgrößenverteilung des mikroporösen Materials sollte zu mindestens 80 %, bevorzugt zu mindestens 90 %, im Bereich der bevorzugten Partikelgröße liegen.

Der Geometrie der Primärpartikel ist keine Beschränkung aufgelegt. Bevorzugt eingesetzt werden Primärpartikel mit einem Aspect-ratio von größer 1, bevorzugt größer 2 und besonderes bevorzugt von größer 5. Der Aspect-ratio ist definiert als das Verhältnis der Länge (in µm) zum Durchmesser (in µm) der Primärpartikel. Die Primärpartikel können vereinzelt oder als Agglomerate bestehend aus minimal zwei, typischerweise zwei bis fünfzig Primärpartikel im Pulver vorliegen.

Die Größe und die Geometrie der Primärpartikel, wie sie im Rahmen der vorliegenden Erfindung beschrieben werden, können beispielsweise über die elektronenmikroskopischen Verfahren SEM (Scanning Electron Microscopy) und TEM (Transmission Electron Microscopy) bestimmt werden. Die Größenverteilung der Primärpartikel kann beilspielsweise durch Messung der Partikelgrößenverteilung mittels Laserbeugung bestimmt werden.

Die spezifische Oberfläche des bevorzugten kristallinen zeolithischen Materials, bestimmt gemäß DIN 66131 (BET), liegt bevorzugt bei mindestens 200 m²/g und insbesondere bevorzugt bei mindestens 300 m²/g. Beispielsweise liegt die spezifische Oberfläche im Bereich von 200 bis 650 m²/g und insbesondere im Bereich von 300 bis 550 m²/g_{.}

Das Porenvolumen des bevorzugten kristallinen zeolithischen Materials, bestimmt gemäß DIN 66134 (Langmuir; p/pₒ = 0,9995), liegt bevorzugt bei mindestens 0,5 ml/g, besonders bevorzugt bei mindestens 0,6 ml/g und insbesondere bevorzugt bei mindestens 0,75 ml/g. Beispielsweise liegt das Porenvolumen im Bereich von 0,5 bis 1,5 ml/g, weiter bevorzugt im Bereich von 0,6 bis 1,4 ml/g und insbesondere bevorzugt im Bereich von 0,75 bis 1,3 ml/g.

### Das Lösungsmittel (Schritt I)

Als Lösungsmittel eignen sich z.B. acyclische oder cyclische, besonders aliphatische, Ether mit 2 bis 12 Kohlenstoffatomen, wie Diethylether, Di-n-propylether oder dessen Isomere, Methyl-tert.-butylether (MTBE), THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art.

Besonders bevorzugt wird als Lösungsmittel, das auch ein Verdünnungsmittel sein kann, Wasser eingesetzt.

Dem Wasser können sowohl Brönsted-Säuren als auch Brönstedt-Basen zugesetzt werden.
Geeignete Brönstedt-Säuren sind beispielsweise Salzsäure, Schwefelsäure, Phosphorsäure, Salpetersäure oder Carbonsäuren, Dicarbonsäuren oder Oligo- oder Polycarbonsäuren wie beispielsweise Nitrilotriessigsäure, Sulfosalicylsäure oder Ethylendiaminotetraessigsäure.
Geeignete Brönstedt-Basen sind primäre, sekundäre und tertiäre Alkylamine, Ammoniak, sowie Seltenerd-, Alkali- und Erdalkalimetallhydroxide.
Der Anteil an Brönstedt-Säuren bzw. Brönstedt-Basen im Lösungsmittel (z.B. Wasser) liegt besonders zwischen 0,5 und 50 Gew.%, bevorzugt zwischen 1 und 25 Gew.%, besonderes bevorzugt zwischen 1,5 und 10 Gew.-%.

Die Zugabe des Lösungsmittels bewirkt, dass das Gemisch die richtige Konsistenz für die Weiterverarbeitung im Formungsschritt hat. Der Anteil an Lösungsmittel liegt bevorzugt im Bereich von 0,5 bis 80 Gew.-%, weiter bevorzugt im Bereich von 1 bis 50 Gew.-%, weiter bevorzugt im Bereich von 2 bis 40 Gew.-%, und besonders bevorzugt im Bereich von 3 bis 30 Gew.-%, jeweils bezogen auf die Gesamtmasse des Gemisches gemäß Schritt I.

### Das Anteigmittel (Schritt I)

Bei der Herstellung des Gemisches gemäß (I) wird mindestens ein Anteigmittel (= organischer Zusatzstoff) zugegeben.

Als Zusatzstoff (= Anteigmittel) können alle dafür geeigneten Verbindungen verwendet werden. Vorzugsweise sind dies organische, insbesondere hydrophile Polymere wie beispielsweise Cellulose, Cellulosederivate, wie beispielsweise Methylcellulose, Stärke, wie beispielsweise Kartoffelstärke, Polyacrylate, Polymethacrylate, Polyvinylalkohol, Polyvinylpyrrolidon (PVP), Polyisobuten (PIB) oder Polytetrahydrofuran (PTHF).

Insbesondere können als Anteigmittel Verbindungen eingesetzt werden, die auch als Porenbildner wirken.

Das Anteigmittel wird bevorzugt als Feststoff eingesetzt.

Das Anteigmittel wird in einer besonders bevorzugten Ausführungsform des erfindungsgemäßen Verfahrens, wie untenstehend beschrieben, im Schritt V durch Calcinieren zu mindestens 90 Gew.-% entfernt.

Die Zugabe des Anteigmittels bewirkt, dass das Gemisch die richtige Konsistenz für die Weiterverarbeitung im Formungsschritt hat. Der Anteil an Anteigmittel liegt bevorzugt im Bereich von 0,5 bis 80 Gew.%, weiter bevorzugt im Bereich von 1 bis 50 Gew.-%, weiter bevorzugt im Bereich von 2 bis 40 Gew.%, und besonders bevorzugt im Bereich von 3 bis 30 Gew.-%, jeweils bezogen auf die Gesamtmasse des Gemisches gemäß Schritt I.

### Porenbildner (optional, Schritt I)

Das Gemisch aus Bindemittel, dem mikroporösen, insbesondere zeolithischen Material, Anteigmittel und Lösungsmittel gemäß I kann zur weiteren Verarbeitung und zur Ausbildung einer plastischen Masse mit mindestens einer weiteren Verbindung versetzt werden. Unter anderem bevorzugt sind hier Porenbildner zu nennen.

Als Porenbildner können im erfindungsgemäßen Verfahren sämtliche Verbindungen eingesetzt werden, die bezüglich des fertigen Formkörpers eine bestimmte Porengröße, eine bestimmte Porengrößenverteilung und/oder bestimmte Porenvolumina bereitstellt.

Bevorzugt werden als Porenbildner im erfindungsgemäßen Verfahren Polymere eingesetzt, die in Wasser oder in wässrigen Lösungsmittelgemischen dispergier-, suspendier- und/oder emulgierbar sind. Bevorzugte Polymere sind hierbei polymere Vinylverbindungen wie beispielsweise Polyalkylenoxide, wie Polyethylenoxide, Polystyrol, Polyacrylate, Polymethacrylate, Polyolefine, Polyamide und Polyester, Kohlenhydrate, wie Cellulose oder Cellulosederivate, wie beispielsweise Methylcellulose, oder Zucker oder Naturfasern. Weitere geeignete Porenbildner sind Pulp oder Graphit.

Bevorzugt sind auch organische saure Verbindungen, die sich in dem Schritt V, wie untenstehend beschrieben, durch Calcinieren entfernen lassen. Zu nennen sind hier Carbonsäuren, insbesondere C₁₋₈-Carbonsäuren, wie beispielsweise Ameisensäure, Oxalsäure und/oder Zitronensäure. Ebenso ist es möglich, zwei oder mehr dieser sauren Verbindungen einzusetzen.

Werden bei der Herstellung des Gemischs gemäß Schritt 1 Porenbildner eingesetzt, so liegt der Gehalt des Gemischs gemäß (I) an Porenbildner bevorzugt im Bereich von 0,5 bis 80 Gew.-%, bevorzugt im Bereich von 1 bis 50 Gew.-% und besonders bevorzugt im Bereich von 2 bis 30 Gew.-%, jeweils bezogen auf die Menge an mikroporösen, insbesondere zeolithischem Material im Gemisch gemäß (I).

Sollte dies für die zu erzielende Porengrößenverteilung erwünscht sein, kann auch ein Gemisch aus zwei oder mehr Porenbildnern eingesetzt werden.

Die Porenbildner werden in einer besonders bevorzugten Ausführungsform des erfindungsgemäßen Verfahrens, wie untenstehend beschrieben, im Schritt V durch Calcinieren unter Erhalt des porösen Formkörpers zu mindestens 90 Gew.-% entfernt. Gemäß einer bevorzugten Ausführungsform des erfindungsgemäßen Verfahrens werden dabei Formkörper erhalten, die Porenvolumina, bestimmt gemäß DIN 66134, im Bereich von mindestens 0,3 ml/g, bevorzugt im Bereich von 0,4 bis 1,0 ml/g und insbesondere bevorzugt im Bereich von mehr als 0,4 ml/g bis 0,8 ml/g aufweisen.

Die spezifische Oberfläche des resultierenden im erfindungsgemäßen Verfahren eingesetzten Formkörpers, bestimmt gemäß DIN 66131, liegt bevorzugt bei mindestens 200 m²/g und insbesondere bei mindestens 250 m²/g.
Beispielsweise liegt die spezifische Oberfläche im Bereich von 200 bis 550 m²/g und bevorzugt im Bereich von 250 bis 500 m²/g.

### Mischen und Verdichten (Schritt II)

Nach der Herstellung des Gemisches gemäß (I) wird das Gemisch homogenisiert, z.B. für eine Dauer im Bereich von 10 bis 180 Minuten. Besonders bevorzugt werden zur Homogenisierung unter anderem Kneter, Koller oder Extruder eingesetzt. Im kleineren Maßstab wird das Gemisch bevorzugt geknetet. Im industriellen, größeren Maßstab wird zur Homogenisierung bevorzugt gekollert.

Bei der Homogenisierung wird bevorzugt bei Temperaturen im Bereich von ungefähr 10°C bis zum Siedepunkt des Lösungsmittels und Normaldruck oder leichtem überatmosphärischem Druck gearbeitet. Danach kann gegebenenfalls mindestens eine der oben beschriebenen Verbindungen zugegeben werden. Das so erhaltene Gemisch wird solange homogenisiert, vorzugsweise geknetet, bis eine verstrangbare plastische Masse entstanden ist.

Das homogenisierte Gemisch wird in einem folgenden Schritt verformt.

### Verformen des verdichteten Gemischs unter Erhalt eines Formkörpers (Schritt III)

Zur Durchführung dieses Schritts sind solche Verfahren bevorzugt, bei denen die Verformung durch Extrusion in üblichen Extrudern, beispielsweise zu Strängen mit einem Durchmesser von bevorzugt 1 bis 10 mm und besonders bevorzugt 2 bis 5 mm, erfolgt. Derartige Extrusionsvorrichtungen werden beispielsweise in Ullmann's Enzyklopädie der Technischen Chemie, 4. Auflage, Bd. 2, S. 295 ff., 1972, beschrieben.

Neben der Verwendung eines Extruders wird ebenfalls vorzugsweise eine Strangpresse zur Verformung verwendet.

Prinzipiell können jedoch zur Formgebung alle bekannten und/oder geeigneten Knet- und Verformungsvorrichtungen bzw. Verfahren eingesetzt werden. Unter anderem sind hierbei zu nennen:
(i) Brikettieren, d.h. mechanisches Verpressen mit oder ohne Zusatz von zusätzlichem Bindermaterial;
(ii) Pelletieren, d.h. Kompaktieren durch kreisförmige und/oder rotierende Bewegungen;
(iii) Sintern, d.h. das zu verformende Material wird einer thermischen Behandlung ausgesetzt.

Beispielsweise kann die Formgebung aus der folgenden Gruppe ausgewählt sein, wobei die Kombination von mindestens zwei dieser Methoden explizit eingeschlossen ist: Brikettieren durch Stempelpressen, Walzenpressen, Ringwalzenpressen, Brikettieren ohne Bindemittel; Pelletieren, Schmelzen, Spinning-Techniken, Abscheidung, Schäumen, Sprühtrocknen; Brennen im Schachtofen, Konvektionsofen, Wanderrost, Drehrohrofen, Kollern.

Das Kompaktieren kann bei Umgebungsdruck oder bei gegenüber dem Umgebungsdruck erhöhtem Druck stattfinden, beispielsweise in einem Druckbereich von 1 bar bis zu mehreren hundert bar. Weiterhin kann das Kompaktieren bei Umgebungstemperatur oder bei gegenüber der Umgebungstemperatur erhöhter Temperatur stattfinden, beispielsweise in einem Temperaturbereich von 20 bis 300 °C. Ist Trocknen und/oder Brennen Bestandteil des Formgebungsschritts, so sind Temperaturen bis zu 1500 °C denkbar. Schließlich kann das Kompaktieren in der Umgebungs-Atmosphäre stattfinden oder in einer kontrollierten Atmosphäre. Kontrollierte Atmosphären sind beispielsweise Schutzgas-Atmosphären, reduzierende und/oder oxidierende Atmosphären.

Die Form der erfindungsgemäß hergestellten Formkörper kann beliebig gewählt werden. Insbesondere sind unter anderem Kugeln, ovale Formen, Zylinder oder Tabletten möglich.

Besonders bevorzugt wird im Rahmen der vorliegenden Erfindung das Verformen durch Extrusion des gemäß Schritt II erhaltenen Gemischs durchgeführt, wobei als Extrudate weiter bevorzugt im wesentlichen zylinderförmige Stränge mit einem Durchmesser im Bereich von 0,5 bis 20 mm, bevorzugt im Bereich von 1 bis 10 mm erhalten werden.

Bei den Extrudaten beträgt das Länge : Durchmesser - Verhältnis vorteilhaft mindestens 0,7, insbesondere mindestens 1, bevorzugt im Bereich von größer 1 bis 20, besonders bevorzugt im Bereich von 2 bis 10.

### Trocknen des Formkörpers (Schritt IV)

Dem Schritt (III) schließt sich im Rahmen der vorliegenden Erfindung bevorzugt mindestens ein Trocknungsschritt an. Dieser mindestens eine Trocknungsschritt erfolgt dabei bei Temperaturen im Bereich von bevorzugt 80 bis 160 °C, insbesondere von 90 bis 145 °C und besonders bevorzugt von 100 bis 130 °C, wobei die Trocknungsdauer bevorzugt bei 6 h oder mehr, beispielsweise im Bereich von 6 bis 24 h, liegt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Vor und/oder nach dem Trocknungsschritt kann das bevorzugt erhaltene Extrudat beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

### Calcinierung des Formkörpers (Schritt V)

Dem Schritt (IV) folgt mindestens ein Calcinierungsschritt. Die Calcinierung wird bei Temperaturen im Bereich von bevorzugt 350 bis 750 °C und insbesondere von 450 bis 700 °C durchgeführt.

Die Calcinierung kann unter jeder geeigneten Gasatmosphäre erfolgen, wobei Luft und/oder Magerluft bevorzugt sind.
Das Calcinieren gemäß (V) kann auch in Gegenwart von Wasserstoff, Stickstoff, Helium, Argon und/oder Dampf oder Gemischen hiervon erfolgen.

Weiter wird die Calcinierung bevorzugt in einem Muffelofen, einem Drehrohrofen und/oder einem Bandkalzinierofen durchgeführt, wobei die Calcinierungsdauer bevorzugt bei 1 h oder mehr, beispielsweise im Bereich von 1 bis 24 h oder im Bereich von 3 bis 12 h liegt. Demgemäß ist es im Rahmen des erfindungsgemäßen Verfahrens beispielsweise möglich, den Formkörper einmal, zweimal oder öfter für jeweils mindestens 1 h, wie beispielsweise jeweils im Bereich von 3 bis 12 h, zu calcinieren, wobei die Temperaturen während eines Calcinierungsschrittes gleich bleiben oder kontinuierlich oder diskontinuierlich geändert werden können. Wird zweimal oder öfter calciniert, können sich die Calcinierungstemperaturen in den einzelnen Schritten unterscheiden oder gleich sein.

Nach dem Calcinierungsschritt kann das calcinierte Material beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

Die erhaltenen Formkörper weisen Härten auf, die bevorzugt im Bereich von 2 bis 150 N (Newton), besonders bevorzugt im Bereich von 5 bis 100 N und ganz besonders bevorzugt mindestens 10 N betragen, z.B. im Bereich von 10 bis 75 N liegen.

Die obenstehend beschriebene Härte wurde im Rahmen der vorliegenden Erfindung an einem Apparat der Firma Zwick, Typ BZ2.5/TS1S mit einer Vorkraft von 0,5 N, einer Vorkraftschubgeschwindigkeit von 10 mm/Min. und einer nachfolgenden Prüfgeschwindigkeit von 1,6 mm/Min, bestimmt. Das Gerät besaß einen festsitzenden Drehteller und einen frei beweglichen Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller hin, auf der der zu untersuchende Katalysatorformkörper lag. Das Prüfgerät wurde über einen Computer gesteuert, der die Messergebnisse registrierte und auswertete. Die erzielten Werte stellen den Mittelwert aus den Messungen zu jeweils 10 Katalysatorformkörpern dar.

Nach dem Calcinieren (Schritt V) kann der Formkörper optional mit einer konzentrierten oder verdünnten Broenstedt-Säure oder einem Gemisch aus zwei oder mehr Broenstedt-Säuren behandelt werden. Geeignete Säuren sind beispielsweise Salzsäure, Schwefelsäure, Phosphorsäure, Salpetersäure oder Carbonsäuren, Dicarbonsäuren oder Oligo- oder Polycarbonsäuren, wie beispielsweise Nitrilotriessigsäure, Sulfosalicylsäure oder Ethylendiaminotetraessigsäure.

Diese Behandlung erfolgt in der, optional wässrigen, Flüssigphase bei einer bevorzugten Temperatur im Bereich 10 bis 120 °C und einer bevorzugten Dauer im Bereich von 0,5 bis 12 h.

Bevorzugt schließt sich an diese mindestens eine Behandlung mit mindestens einer Broenstedtsäure mindestens ein Trockenschritt und/oder mindestens ein Calcinierungsschritt an, der jeweils unter den oben beschriebenen Bedingungen durchgeführt wird.

Gemäß einer weiteren bevorzugten Ausführungsform des erfindungsgemäßen Verfahrens können die Katalysatorstränge zur besseren Härtung einer Wasserdampfbehandlung unterzogen werden, nach der bevorzugt nochmals mindestens einmal getrocknet und/oder mindestens einmal calciniert wird. Beispielsweise wird nach mindestens einem Trockenschritt und mindestens einem nachfolgenden Calcinierschritt der calcinierte Formkörper der Wasserdampfbehandlung unterzogen und anschließend nochmals mindestens einmal getrocknet und/oder mindestens einmal calciniert.

Verwendung der Katalysatorformkörper im Verfahren zur kontinuierlichen Synthese von Methylaminen durch Umsetzung von Methanol und/oder Dimethylether mit Ammoniak:

Die Herstellung von Methylaminen an dem Katalysator des erfindungsgemäßen Verfahrens erfolgt durch Umsetzung von Ammoniak und Methanol und/oder Dimethylether in der Gasphase bei erhöhtem Druck und erhöhter Temperatur. Wahlweise kann dem/das Reaktionsgemisch Wasser, Monomethylamin, Dimethylamin und/oder Trimethylamin zugesetzt werden bzw. enthalten.

Die Katalysatorbelastung, ausgedrückt in Kilogramm Methanol pro Kilogramm Katalysator pro Stunde, liegt bevorzugt im Bereich von 0,1 bis 2,0 h⁻¹, insbesondere im Berich von 0,2 bis 1,5 h⁻¹, ganz besonders im Bereich von 0,4 bis 1,0 h⁻¹.

Das molare N/C-Verhältnis bezogen auf die Summe der Einsatzstoffe liegt bevorzugt im Bereich von 0,6 bis 4,0, insbesondere 0,8 bis 2,5, ganz besonders 1,0 bis 2,0.

Die Umsetzung wird bevorzugt bei einer Temperatur im Bereich von 250 bis 450 °C, besonders 280 bis 350 °C, ganz besonders 290 bis 330 °C, durchgeführt.

Der Absolutdruck der Umsetzung liegt bevorzugt im Bereich von 5 bis 50 bar, besonders 10 bis 30 bar, insbesondere 15 bis 25 bar.

Der Umsatz an Methanol liegt bevorzugt bei ≥ 85 %, besonderes bevorzugt zwischen 90 % und 99 %, insbesondere zwischen 90 % und 95 %.

Die Selektivität der Umsetzung bezüglich Mono-, Di- und Trimethylamin liegt bevorzugt bei ≥ 95 %, besonderes bevorzugt bei ≥ 98 %.

Mit dem erfindungsgemäßen Verfahren fallen die Methylamine bevorzugt im Gewichtsverhältnis Monomethylamin (MMA) : Dimethylamin (DMA) : Trimethylamin (TMA) = < 35 : > 55 : ≤ 10, insbesondere MMA: DMA: TMA = ≤ 35 : ≥ 60 : ≤ 5, an.

Besonders bevorzugt erfolgt die Umsetzung unter isothermen Bedindungen, d.h. bei einer Abweichung von maximal +/- 20 °C, bevorzugt +/- 5 °C, besonders bevorzugt +/- 10 °C, insbesondere +/- 5 °C, ganz besonders +/- 4 °C, der vorgegebenen Reaktionstemperatur.

Geeignete Reaktoren hierfür sind Rohrbündelreaktoren oder Isothermreaktoren, wie sie z.B. in DE-A-34 14 717 (Linde AG, ,Lindereaktor'), in EP-A1-534 195 (BASF AG) und in WO-A1-04/048313 (BASF AG) für die Methylamine-Synthese beschrieben sind, oder adiabate Reaktoren mit Zwischenkühlung.

Die Aufarbeitung des Reaktoraustrags kann in Anlehnung an dem Fachmann bekannten Verfahren, z.B. gemäß DD-125 533 (VEB Leuna-Werke), erfolgen.

Für das Verfahren zur Herstellung von Methylaminen mit dem erfindungsgemäßen formselektiven Katalysator ist die Verschaltung mit einem Reaktor enthaltend einen Gleichgewichtskatalysator gemäß US 4,485,261 und PEP-Review, No. 89-3-4 bevorzugt.

Um eine lange Laufzeit des formselektiven Katalysators zu gewährleisten, sollte der Anteil an Aldehyden und insbesondere der Anteil an Formaldehyd im Feed bevorzugt weniger als 0,15 g pro kg Katalysator pro Stunde betragen (vergl. EP-A-342 999).

Bei dem gegebenenfalls eingesetzten Dimethylether (DME), Trimethylamin (TMA) und/oder dem gegebenenfalls eingesetzten Monomethylamin (MMA) handelt es sich in besonderen Ausgestaltungen der Erfindung um einen jeweiligen Rückführstrom aus dem aufgearbeiteten Reaktionsprodukt des Verfahrens.

Regenerierung der Katalysatorformkörper nach der kontinuierlichen Synthese von Methylaminen durch Umsetzung von Methanol und/oder Dimethylether mit Ammoniak:

In einer weiteren Ausführungsform des erfindungsgemäßen Verfahrens wird der Katalysator nach dem Einsatz unabhängig von seiner Form, z.B. nach Abnahme der Aktivität und/oder der Selektivität, durch ein Verfahren regeneriert, bei dem die Regenerierung durch gezieltes Abbrennen (bei z.B. einer Temperatur im Bereich von 350 bis 650 °C) der für die Deaktivierung verantwortlichen Beläge erfolgt. Dabei wird bevorzugt in einer Inertgasatmosphäre gearbeitet, die genau definierte Mengen an Sauerstoff oder Sauerstoff liefernden Substanzen enthält. Ein solches Regenerierungsverfahren ist unter anderem in der WO-A-98/55228 und der DE-A1-197 23 949 und insbesondere für Katalysatoren zur Herstellung von Methylaminen in der JP-08 157 428 und der EP-A-0118 193 beschrieben, deren diesbezügliche Offenbarung durch Bezugnahme hiermit vollumfänglich in den Gegenstand der vorliegenden Anmeldung einbezogen wird.

Nach der Regeneration sind die Aktivität und/oder die Selektivität des Katalysators, verglichen mit dem Zustand unmittelbar vor der Regeneration, erhöht.

Der zu regenerierende, erfindungsgemäß eingesetzte Zeolith-Katalysator wird entweder in der Umsetzungsvorrichtung (Reaktor) oder in einem externen Ofen in einer Atmosphäre, die 0,1 bis ungefähr 20 Volumen-Anteile von Sauerstoff liefernden Substanzen, besonders bevorzugt 0,1 bis 20 Volumenanteile Sauerstoff, enthält, auf eine Temperatur im Bereich von 250 °C bis 800 °C, vorzugsweise von 400 °C bis 650 °C und insbesondere von 425 °C bis 500 °C aufgeheizt. Dabei wird das Aufheizen vorzugsweise mit einer Aufheizrate von 0,1 °C/Min. bis 20 °C/Min., vorzugsweise von 0,3 °C/Min. bis 15 °C/Min. und insbesondere von 0,5 °C/Min. bis 10 °C/Min. durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators.

Sinkt die Temperatur des Abgasstroms am Reaktorausgang trotz steigender Mengen an Sauerstoff liefernden Substanzen im Gasstrom und/oder steigt die Konzentration an Sauerstoff im Reaktionsaustrag auf den Eingangswert an, so ist das Abbrennen der organischen Beläge beendet. Die Dauer der Behandlung beträgt bevorzugt jeweils 1 bis 30 h, vorzugsweise ungefähr 2 bis ungefähr 20 h und insbesondere ungefähr 3 bis ungefähr 10 Stunden.

Das anschließende Abkühlen des so regenerierten Katalysators wird bevorzugt so durchgeführt, dass das Abkühlen nicht zu schnell erfolgt, da sonst die mechanische Festigkeit des Katalysators negativ beeinflusst werden kann.

Es kann erforderlich sein, den Katalysator nach der durchgeführten Regeneration durch Calcinieren, wie oben beschrieben, einer Spülung mit Wasser und/oder verdünnten Säuren wie beispielsweise Salzsäure zu unterziehen, um die durch Verunreinigung der Edukte gegebenenfalls verbleibende anorganische Beladung des Katalysators (Alkalispuren etc.) zu entfernen. Anschließend kann eine erneute Trocknung und/oder ein erneutes Calcinieren des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im Allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere in den Mikroporen zu vermeiden, da auch dies zu Schädigungen des Katalysators führen kann.

Eine bevorzugte Ausführung des Herstellverfahrens besteht darin, dass das erfindungsgemäße, kontinuierliche Verfahren zur Synthese von Methylaminen bei der Regeneration des erfindungsgemäßen Katalysators nicht unterbrochen werden muss, um so den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung des Eduktes oder der Edukte zur Verfügung steht.

### Beispiele

Die BET-Oberflächen (m²/g) und die Porenvolumina (ml/g) wurden nach DIN 66131 bzw. DIN 66134 Norm bestimmt.

### GC-Analytik:

Die Reaktionsausträge wurden mittels Online-Gaschromatographie analysiert. Die Trennung der Methylamine erfolgte dabei auf einer für kurzkettige Amine optimierten GC-Säule (Varian CP-Volamine), zur Detektion wurde ein Wärmeleitfähigkeitsdetektor (WLD) eingesetzt. Der Gehalt an nicht umgesetzten Methanol wurde bestimmt, woraus auf die Aktivität des Katalysators geschlossen wurde.

Die Bestimmung/Messung der Schneidhärte erfolgte wie in der WO-A-04/108280 (BASF AG) beschrieben:
Die Schneidhärten wurden gemessen an einem Apparat der Firma Zwick (Typ: BZ2.5/TS1S; Vorkraft: 0,5 N, Vorkraft Geschwindigkeit: 10 mm/Min.; Prüfgeschwindigkeit: 1,6 mm/Min.) und sind die Mittelwerte von jeweils 10 gemessenen Katalysatorsträngen.

Die Schneidhärte wurde im Detail wie folgt bestimmt (siehe auch weiter oben in der Beschreibung):
Extrudate wurden durch eine Schneide von 0,3 mm Stärke mit zunehmender Kraft belastet, bis das Extrudat durchtrennt war. Die dazu benötigte Kraft ist die Schneidehärte in N (Newton). Die Bestimmung erfolgte auf einem Prüfgerät der Firma Zwick, Ulm, mit festsitzendem Drehteller und frei beweglichem, vertikalem Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller, auf der das zu messende Extrudat lag. Das Prüfgerät wurde durch einen Computer gesteuert, der die Meßergebnisse registrierte und auswertete. Aus einer gut durchmischten Katalysatorprobe wurden 10 gerade, möglichst rissefreie .Extrudate mit einer mittleren Länge von 2 bis 3 mal dem Durchmesser entnommen, deren Schneidhärten ermittelt und anschließend gemittelt.

### Vergleichsbeispiel 1

150 g synthetisches small-port Mordenit (H-Form, Si/Al = 6,0) wurde zusammen mit 71 ml Wasser, 93 g Ludox® AS40 (= kolloidales Silica) und 7,5 g Methylcellulose in einem mechanischen Kneter 60 Minuten gemischt. Danach wurde die Paste in einer Strangpresse zu 2 mm Stränge verformt. Die Stränge wurden 16 h bei 120°C getrocknet und anschließend in einem Muffelofen 5 h bei 500°C unter Luftzufuhr kalziniert. Man erhielt 164 g Katalysator. Die Schneidhärte der Stränge betrug 2,1 N. Die Katalysatorstränge wurden über ein 1,6 mm Sieb gesplittet und die Fraktion zwischen 1,6 und 0,5 mm gesammelt.

### Beispiel 1

Die Herstellung erfolgte analog zum Vergleichsbeispiel 1 aus 105 g synthetischem small-port Mordenit (H-Form, Si/Al = 6,0), 73 ml Wasser, 37 g Silres® MSE100 (= Methylsilikon in Toluol) und 5,2 g Methylcellulose. Man erhielt 109 g Katalysator. Die Schneidhärte der Stränge betrug 25 N. Die Katalysatorstränge wurden über ein 1,6 mm Sieb gesplittet und die Fraktion zwischen 1,6 und 0,5 mm gesammelt.

### Beispiel 2

Die Herstellung erfolgte analog zum Vergleichsbeispiel 1 aus 150 g synthetischem small-port Mordenit (H-Form, Si/Al = 6,0), 52 ml Wasser, 93 g Ludox® AS40, 11 g Silres® MSE100 und 7,5 g Methylcellulose. Man erhielt 164 g Katalysator. Die Schneidhärte der Stränge betrug 5,4 N. Die Katalysatorstränge wurden über ein 1,6 mm Sieb gesplittet und die Fraktion zwischen 1,6 und 0,5 mm gesammelt.

### Synthesebeispiel 1

In einem elektrisch beheizten Rohrreaktor (Länge 50 cm, Durchmesser 12 mm) wurden 25 ml Katalysator aus Vergleichsbeispiel 1 eingebaut. Der Katalysator wurde unter einem Stickstoffstrom über Nacht bei 320°C ausgeheizt. Danach wurde NH₃ (18,1 g/h) und Methanol (18,9 g/h) bei 20 bar zudosiert. Nach einer Laufzeit von 10 h wurde der Reaktionsaustrag gaschromatographisch untersucht.

Der Methanolumsatz betrug 99,6 %. Der Anteil an Monomethylamin (MMA), Dimethylamin (DMA) und Trimethylamin (TMA) bezogen auf die drei gebildeten Methylamine betrug 29 : 46 : 25 Gew.-%.

### Synthesebeispiel 2

Die Versuchsdurchführung erfolgte analog Synthesebeispiel 1, jedoch mit dem Katalysator aus Beispiel 1. Nach 10 h betrug der Methanolumsatz 98,3 %, der Anteil an MMA : DMA : TMA betrug 34 : 65 : 1 Gew.-% (bezogen auf die drei gebildeten Methylamine).
Nach einer Laufzeit von 30 h betrug der Methanolumsatz 97,5 % und der Anteil an MMA : DMA : TMA betrug 34 : 65 : 1 Gew.-% (bezogen auf die drei gebildeten Methylamine).

### Synthesebeispiel 3

Die Versuchsdurchführung erfolgte analog Synthesebeispiel 1, jedoch mit dem Katalysator aus Beispiel 2. Nach 10 h betrug der Methanolumsatz 98,8 %, der Anteil an MMA : DMA : TMA betrug 33 : 63 : 4 Gew.-% (bezogen auf die drei gebildeten Methylamine).
Nach einer Laufzeit von 30 h betrug der Methanolumsatz 98,4 % und der Anteil an MMA : DMA : TMA betrug 33 : 63 : 4 Gew.-% (bezogen auf die drei gebildeten Methylamine).

## Patentansprüche

1. Verfahren zur kontinuierlichen Synthese von Methylaminen durch Umsetzung von Methanol und/oder Dimethylether mit Ammoniak in Gegenwart eines Heterogenkatalysators, **dadurch gekennzeichnet, dass** als Katalysator ein Formkörper enthaltend ein mikroporöses Material und mindestens eine siliziumorganische Verbindung als Bindemittel, herstellbar durch ein Verfahren umfassend die Schritte
(I) Herstellen eines Gemischs, enthaltend das mikroporöse Material, das Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers
eingesetzt wird.

2. Verfahren nach Anspruch 1, **dadurch gekennzeichnet, dass** im Verfahren zur Herstellung des Katalysatorformkörpers als Bindemittel ein Silikon eingesetzt wird.

3. Verfahren nach Anspruch 1, **dadurch gekennzeichnet, dass** im Verfahren zur Herstellung des Katalysatorformkörpers als Bindemittel ein Methylsilikon eingesetzt wird.

4. Verfahren nach Anspruch 1, **dadurch gekennzeichnet, dass** im Verfahren zur Herstellung des Katalysatorformkörpers als Bindemittel ein cyclisches Silikon der Formel [-SiO(OR)(R')-]ₓ oder ein lineares Silikon der Formel RO-[SiO(OR)(R')-]ₓ-R oder ein Gemisch dieser Silikone eingesetzt wird, wobei R und R' C₁₋₆-Alkylgruppen und x eine Zahl im Bereich von 2 bis 50 bedeuten.

5. Verfahren nach einem der vorhergehenden Ansprüche, **dadurch gekennzeichnet, dass** durch die Calcinierung der Formkörper gemäß (V) mindestens 80 Gew.-% der siliziumorganischen Verbindung in hochdisperses SiO₂ umgewandelt wird und der Gewichtsanteil des so gebildeten hochdispersen SiO₂ im Formkörper im Bereich von 5 bis 70 Gew.-% liegt.

6. Verfahren nach dem vorhergehenden Anspruch, **dadurch gekennzeichnet, dass** der Gewichtsanteil des gebildeten hochdispersen SiO₂ im Formkörper im Bereich von 10 bis 50 Gew.-% liegt.

7. Verfahren nach einem der vorhergehenden Ansprüche, **dadurch gekennzeichnet, dass** das Calcinieren gemäß (V) in Gegenwart von Luft, Wasserstoff, Stickstoff, Helium, Argon und/oder Dampf oder Gemischen hiervon erfolgt.

8. Verfahren nach einem der vorhergehenden Ansprüche, **dadurch gekennzeichnet, dass** im Verfahren zur Herstellung des Katalysatorformkörpers das mikroporöse Material gemäß (I) ein kristallines Silikalit, ein kristallines Alumosilikat (= zeolithisches Material), ein kristallines Silicoalumophosphat und/oder ein kristallines Alumophosphat ist.

9. Verfahren nach dem vorhergehenden Anspruch, **dadurch gekennzeichnet, dass** das kristalline Alumosilikat ein Zeolith ist und den Strukturtyp MOR, CHA, ERI, KFI, RHO, BEA, FAU, OFF, NES, HEU, FER, MFI oder MEL aufweist.

10. Verfahren nach dem vorhergehenden Anspruch, **dadurch gekennzeichnet, dass** der Zeolith ein Mordenit mit small-port Eigenschaften ist.

11. Verfahren nach einem der Ansprüche 8 bis 10, **dadurch gekennzeichnet, dass** das zeolithische Material eine spezifische Oberfläche (gemäß DIN 66131 (BET)) von mindestens 200 m²/g aufweist und Poren mit einem Porenvolumen von mindestens 0,5 ml/g (gemäß DIN 66134 (Langmuir)) enthält.

12. Verfahren nach einem der vorhergehenden Ansprüche, **dadurch gekennzeichnet, dass** es sich im Verfahren zur Herstellung des Katalysatorformkörpers bei dem Lösungsmittel gemäß (I) um Wasser handelt.

13. Verfahren nach einem der vorhergehenden Ansprüche, **dadurch gekennzeichnet, dass** es sich im Verfahren zur Herstellung des Katalysatorformkörpers bei dem Anteigmittel gemäß (I) um Cellulose, ein Cellulosederivat und/oder eine Stärke handelt.

14. Verfahren nach einem der vorhergehenden Ansprüche, **dadurch gekennzeichnet, dass** im Verfahren zur Herstellung des Katalysatorformkörpers das Gemisch gemäß (I) zusätzlich mindestens einen Porenbildner enthält.

15. Verfahren nach dem vorhergehenden Anspruch, **dadurch gekennzeichnet, dass** es sich bei dem Porenbildner um ein Polyalkylenoxid, Polyacrylat, Pulp und/oder Graphit handelt.

16. Verfahren nach einem der vorhergehenden Ansprüche, **dadurch gekennzeichnet, dass** im Verfahren zur Herstellung des Katalysatorformkörpers das Verformen gemäß (III) durch Extrusion erfolgt.

17. Verfahren nach dem vorhergehenden Anspruch, **dadurch gekennzeichnet, dass** der Durchmesser der Extrudate im Bereich von 0,5 bis 20 mm und das Länge- zu Durchmesser-Verhältnis im Bereich von 0,7 bis 10 liegt.

18. Verfahren nach einem der vorhergehenden Ansprüche, **dadurch gekennzeichnet, dass** im Verfahren zur Herstellung des Katalysatorformkörpers das Calcinieren gemäß (V) bei einer Temperatur im Bereich von 350 bis 750 °C und einer Dauer im Bereich von 1 bis 24 h erfolgt.

19. Verfahren nach einem der vorhergehenden Ansprüche, **dadurch gekennzeichnet, dass** der Katalysatorformkörper eine Schneidhärte von mindestens 10 N aufweist.

20. Verfahren nach einem der vorhergehenden Ansprüche, **dadurch gekennzeichnet, dass** der Katalysatorformkörper eine spezifische Oberfläche (gemäß DIN 66131 (BET)) von mindestens 200 m²/g aufweist und Poren mit einem Porenvolumen von mindestens 0,3 ml/g (gemäß DIN 66134 (Langmuir)) enthält.

21. Verfahren nach einem der vorhergehenden Ansprüche, **dadurch gekennzeichnet, dass** das mikroporöse Material im Katalysatorformkörper gemäß Anspruch 1 (I) zumindest teilweise in der H⁺- und/oder NH₄⁺-Form vorliegt.

22. Verfahren zur kontinuierlichen Synthese von Methylaminen nach einem der vorhergehenden Ansprüche, **dadurch gekennzeichnet, dass** der Feedstrom neben Methanol und/oder Dimethylether und Ammoniak Monomethylamin, Dimethylamin und/oder Trimethylamin enthält.

23. Verfahren nach einem der vorhergehenden Ansprüche, **dadurch gekennzeichnet, dass** das molare N/C-Verhältnis im Feedgemisch im Bereich von 0,6 bis 4,0 liegt.

24. Verfahren nach einem der vorhergehenden Ansprüche, **dadurch gekennzeichnet, dass** die Reaktionstemperatur im Bereich von 250 bis 450 °C liegt.

25. Verfahren nach einem der vorhergehenden Ansprüche, **dadurch gekennzeichnet, dass** der Absolutdruck im Bereich von 5 bis 50 bar liegt.

26. Verfahren nach einem der vorhergehenden Ansprüche, **dadurch gekennzeichnet, dass** die Katalysatorbelastung, ausgedrückt in Kilogramm Methanol pro Kilogramm Katalysator pro Stunde, im Bereich von 0,1 bis 2,0 h⁻¹ liegt.

27. Verfahren nach einem der vorhergehenden Ansprüche, **dadurch gekennzeichnet, dass** der Anteil an Trimethylamin (TMA) im Produktgemisch bezogen auf die Summe der Methylamine weniger als 10 Gew.-% beträgt.

28. Verfahren nach einem der vorhergehenden Ansprüche, **dadurch gekennzeichnet, dass** eine Regenerierung des eingesetzten Katalysators durch gezieltes Abbrennen der für die Deaktivierung verantwortlichen Beläge erfolgt.

## Claims

1. A process for the continuous synthesis of methylamines by reaction of methanol and/or dimethyl ether with ammonia in the presence of a heterogeneous catalyst, wherein the catalyst used is a shaped body which comprises a microporous material and at least one organosilicon compound as binder and can be produced by a process comprising the steps
(I) preparation of a mixture comprising the microporous material, the binder, a pasting agent and a solvent,
(II) mixing and densification of the mixture,
(IIT)shaping of the densified mixture to give a shaped body,
(IV)drying of the shaped body and
(V) calcination of the dried shaped body.

2. The process according to claim 1, wherein a silicone is used as binder in the process for producing the shaped catalyst body.

3. The process according to claim 1, wherein a methylsilicone is used as binder in the process for producing the shaped catalyst body.

4. The process according to claim 1, wherein a cyclic silicone of the formula [-SiO(OR) (R')-]ₓ or a linear silicone of the formula RO-[SiO(OR)(R')-]ₓ-R, where R and R' are C₁₋₆-alkyl groups and x is in the range from 2 to 50, or a mixture of these silicones is used as binder in the process for producing the shaped catalyst body.

5. The process according to any of the preceding claims, wherein at least 80% by weight of the organosilicon compound is converted into finely divided SiO₂ by the calcination of the shaped bodies in (V) and the proportion by weight of the resulting finely divided SiO₂ in the shaped body is in the range from 5 to 70% by weight.

6. The process according to the preceding claim, wherein the proportion by weight of the resulting finely divided SiO₂ in the shaped body is in the range from 10 to 50% by weight.

7. The process according to any of the preceding claims, wherein the calcination in (V) is carried out in the presence of air, hydrogen, nitrogen, helium, argon and/or steam or a mixture thereof.

8. The process according to any of the preceding claims, wherein the microporous material used in (I) in the process for producing the shaped catalyst body is a crystalline silicalite, a crystalline aluminosilicate (= zeolitic material), a crystalline silicoaluminophosphate and/or a crystalline aluminophosphate.

9. The process according to the preceding claim, wherein the crystalline aluminosilicate is a zeolite and has a structure of the MOR, CHA, ERI, KFI, RHO, BEA, FAU, OFF, NES, HEU, FER, MFI or MEL type.

10. The process according to the preceding claim, wherein the zeolite is a mordenite having small-port properties.

11. The process according to any of claims 8 to 10, wherein the zeolitic material has a specific surface area (in accordance with DIN 66131 (BET)) of at least 200 m²/g and comprises pores having a pore volume of at least 0.5 ml/g (in accordance with DIN 66134 (Langmuir)).

12. The process according to any of the preceding claims, wherein the solvent used in (I) in the process for producing the shaped catalyst body is water.

13. The process according to any of the preceding claims, wherein the pasting agent used in (I) in the process for producing the shaped catalyst body is cellulose, a cellulose derivative and/or a starch.

14. The process according to any of the preceding claims, wherein the mixture used in (I) in the process for producing the shaped catalyst body further comprises at least one pore former.

15. The process according to the preceding claim, wherein the pore former is a polyalkylene oxide, polyacrylate, pulp and/or graphite.

16. The process according to any of the preceding claims, wherein the shaping in (III) in the process for producing the shaped catalyst body is carried out by extrusion.

17. The process according to the preceding claim, wherein the diameter of the extrudates is in the range form 0.5 to 20 mm and the length-to-diameter ratio is in the range from 0.7 to 10.

18. The process according to any of the preceding claims, wherein the calcination in (V) in the process for producing the shaped catalyst body is carried out at a temperature in the range from 350 to 750°C and for a time in the range from 1 to 24 hours.

19. The process according to any of the preceding claims, wherein the shaped catalyst body has a cutting hardness of at least 10 N.

20. The process according to any of the preceding claims, wherein the shaped catalyst body has a specific surface area (in accordance with DIN 66131 (BET)) of at least 200 m²/g and comprises pores having a pore volume of at least 0.3 ml/g (in accordance with DIN 66134 (Langmuir)).

21. The process according to any of the preceding claims, wherein the microporous material in the shaped catalyst body as set forth in claim 1 (I) is present at least partly in the H⁺ and/or NH₄⁺ form.

22. The process for the continuous synthesis of methylamines according to any of the preceding claims, wherein the feed stream comprises methanol and/or dimethyl ether and ammonia together with monomethylamine, dimethylamine and/or trimethylamine.

23. The process according to any of the preceding claims, wherein the molar N/C ratio in the feed mixture is in the range from 0.6 to 4.0.

24. The process according to any of the preceding claims, wherein the reaction temperature is in the range from 250 to 450°C.

25. The process according to any of the preceding claims, wherein the absolute pressure is in the range from 5 to 50 bar.

26. The process according to any of the preceding claims, wherein the space velocity over the catalyst, expressed in kilograms of methanol per kilogram of catalyst per hour, is in the range from 0.1 to 2.0 h⁻¹.

27. The process according to any of the preceding claims, wherein the proportion of trimethylamine (TMA.) in the product mixture based on the sum of the methylamines is less than 10% by weight.

28. The process according to any of the preceding claims, wherein a regeneration of the catalyst used is carried out by targeted burning-off of the deposits responsible for deactivation.

## Revendications

1. Procédé pour la synthèse continue de méthylamines par mise en réaction de méthanol et/ou d'éther diméthylique avec de l'ammoniac en présence d'un catalyseur hétérogène, **caractérisé en ce qu'**on utilise comme catalyseur un corps moulé contenant une matière microporeuse et au moins un composé organosilicié en tant que liant, pouvant être produit par un procédé comprenant les étapes
(I) préparation d'un mélange, contenant la matière microporeuse, le liant, un agent de mise en pâte et un solvant,
(II) mélange et compactage du mélange,
(III) mise en forme du mélange compacté, avec obtention d'un corps moulé,
(IV) séchage du corps moulé et
(V) calcination du corps moulé séché.

2. Procédé selon la revendication 1, **caractérisé en ce que** dans le procédé pour la production du corps moulé catalyseur on utilise comme liant un silicone.

3. Procédé selon la revendication 1, **caractérisé en ce que** dans le procédé pour la production du corps moulé catalyseur on utilise comme liant un méthylsilicone.

4. Procédé selon la revendication 1, **caractérisé en ce que** dans le procédé pour la production du corps moulé catalyseur on utilise comme liant un silicone cyclique de formule [-SiO(OR)(R')-]ₓ ou un silicone linéaire de formule RO-[SiO(OR)(R')-]ₓ-R ou un mélange de ces silicones, R et R' représentant des groupes alkyle en C₁-C₆ et x représentant un nombre dans la plage de 2 à 50.

5. Procédé selon l'une quelconque des revendications précédentes, **caractérisé en ce que** par la calcination du corps moulé selon (V) au moins 80 % du composé organosilicié sont convertis en SiO₂ hautement dispersé et la proportion pondérale du SiO₂ hautement dispersé, ainsi formé, dans le corps moulé se situe dans la plage allant de 5 à 70 % en poids ;

6. Procédé selon la revendication précédente, **caractérisé en ce que** la proportion pondérale du SiO₂ hautement dispersé, formé, dans le corps moulé se situe dans la plage allant de 10 à 50 % en poids.

7. Procédé selon l'une quelconque des revendications précédentes, **caractérisé en ce que** la calcination selon (V) s'effectue en présence d'air, d'hydrogène, d'azote, d'hélium, d'argon et/ou de vapeur d'eau ou de mélanges de ceux-ci.

8. Procédé selon l'une quelconque des revendications précédentes, **caractérisé en ce que** dans le procédé pour la production du corps moulé catalyseur, la matière microporeuse selon (I) est une silicalite cristalline, un aluminosilicate cristallin (= matière zéolithique), un silico-aluminophosphate cristallin et/ou un aluminophosphate cristallin.

9. Procédé selon l'une quelconque des revendications précédentes, **caractérisé en ce que** l'aluminosilicate cristallin est une zéolithe et présente le type de structure MOR, CHA, ERI, KFI, RHO, BEA, FAU, OFF, NES, HEU, FER, MFI ou MEL.

10. Procédé selon l'une quelconque des revendications précédentes, **caractérisé en ce que** la zéolithe est une mordénite à propriétés de type small-port.

11. Procédé selon l'une quelconque des revendications 8 à 10, **caractérisé en ce que** la matière zéolithique présente une surface spécifique [selon DIN 66131 (BET)] d'au moins 200 m²/g et contient des pores ayant un volume de pore d'au moins 0,5 ml/g [selon DIN 66134 (Langmuir)].

12. Procédé selon l'une quelconque des revendications précédentes, **caractérisé en ce que** dans le procédé pour la production du corps moulé catalyseur le solvant selon (I) consiste en eau.

13. Procédé selon l'une quelconque des revendications précédentes, **caractérisé en ce que** dans le procédé pour la production du corps moulé catalyseur l'agent de mise en pâte selon (I) consiste en cellulose, en un dérivé de cellulose et/ou en un amidon.

14. Procédé selon l'une quelconque des revendications précédentes, **caractérisé en ce que** dans le procédé pour la production du corps moulé catalyseur le mélange selon (I) contient additionnellement au moins un agent porogène.

15. Procédé selon la revendication précédente, **caractérisé en ce que** l'agent porogène consiste en un polyoxyalkylène, un polyacrylate, de la pulpe et/ou du graphite.

16. Procédé selon l'une quelconque des revendications précédentes, **caractérisé en ce que** dans le procédé pour la production du corps moulé catalyseur la mise en forme selon (III) s'effectue par extrusion.

17. Procédé selon la revendication précédente, **caractérisé en ce que** le diamètre de l'extrudat se situe dans la plage de 0,5 à 20 mm et le rapport de la longueur au diamètre de situe dans la plage de 0,7 à 10.

18. Procédé selon l'une quelconque des revendications précédentes, **caractérisé en ce que** dans le procédé pour la production du corps moulé catalyseur la calcination selon (V) s'effectue à une température dans la plage de 350 à 750 °C et pendant une durée dans la plage de 1 à 24 heures.

19. Procédé selon l'une quelconque des revendications précédentes, **caractérisé en ce que** le corps moulé catalyseur présente une dureté à la coupe d'au moins 10 N.

20. Procédé selon l'une quelconque des revendications précédentes, **caractérisé en ce que** le corps moulé catalyseur présente une surface spécifique [selon DIN 66131 (BET)] d'au moins 200 m²/g et contient des pores ayant un volume de pore d'au moins 0,3 ml/g [selon DIN 66134 (Langmuir)].

21. Procédé selon l'une quelconque des revendications précédentes, **caractérisé en ce que** la matière microporeuse dans le corps moulé catalyseur selon la revendication 1 (I) se trouve au moins en partie sous la forme H⁺ et/ou NH₄⁺.

22. Procédé pour la synthèse continue de méthylamines selon l'une quelconque des revendications précédentes, **caractérisé en ce qu'**en plus de méthanol et/ou d'éther diméthylique et d'ammoniac le courant d'addition contient de la monoéthanolamine, de la diméthylamine et/ou de la triéthylamine.

23. Procédé selon l'une quelconque des revendications précédentes, **caractérisé en ce que** le rapport molaire N/C dans le mélange d'addition se situe dans la plage de 0,6 à 4,0.

24. Procédé selon l'une quelconque des revendications précédentes, **caractérisé en ce que** la température de la réaction se situe dans la plage allant de 250 à 450 °C.

25. Procédé selon l'une quelconque des revendications précédentes, **caractérisé en ce que** la pression absolue se situe dans la plage allant de 5 à 50 bars.

26. Procédé selon l'une quelconque des revendications précédentes, **caractérisé en ce que** la charge du catalyseur, exprimée en kilogramme de méthanol par kilogramme de catalyseur par heure, se situe dans la plage allant de 0,1 à 2,0 h⁻¹.

27. Procédé selon l'une quelconque des revendications précédentes, **caractérisé en ce que** la proportion de triméthylamine (TMA) dans le mélange produit, par rapport à la somme des méthylamines, est inférieure à 10 % en poids.

28. Procédé selon l'une quelconque des revendications précédentes, **caractérisé en ce qu'**une régénération du catalyseur utilisé s'effectue par brûlage intentionnel des dépôts responsables de l'inactivation.
